# DEMANDE DE BREVET EUROPEEN

(11) **EP 2 586 476 A2**
(43) Date de publication de la demande: **01.05.2013**
(21) Numéro de dépôt: 12190658.0
(22) Date de dépôt: 30.10.2012
(51) Int. Cl.: A61M 5/145, A61M 5/315, A61M 1/00, A61M 5/20, A61M 39/16, A61M 39/00, A61M 5/24, A61B 17/00, A61M 39/18

(54) **Dispositif de prélèvement et de réinjection de tissu adipeux, injecteur et nécessaire de prélèvement associés**

(30) Priorité: 31.10.2011 FR 1159876
(71) Demandeur: Symatese, 69630 Chanopost (FR)
(72) Inventeur: Perouse, Eric, 75016 PARIS (FR)
(74) Mandataire: Blot, Philippe Robert Emile

(57) **Abrégé**

Ce dispositif comporte un corps creux (22) délimitant une cavité (40) de réception du fluide, la cavité de réception (40) débouchant par une ouverture proximale (46) et par au moins une ouverture distale (48) de prélèvement et/ou de réinjection et un piston (24), monté mobile dans la cavité de réception (40) pour obturer l'ouverture proximale (46).

Le dispositif comporte un ensemble de distribution (26) sélective d'une partie proximale du fluide située dans la cavité de réception (40), l'ensemble de distribution (26) comportant un canal (70) de circulation de fluide ménagé à travers le piston (24), le canal de circulation (70) débouchant dans la cavité de réception (40). Le canal de circulation (76) et/ou un canal de distribution (48) est apte à coopérer ou communiquer avec un dispositif (312) de régulation de débit pouvant être actionné manuellement.

## Description

La présente invention concerne un dispositif de prélèvement et de réinjection d'un fluide, du type comprenant :
- un corps creux délimitant une cavité de réception du fluide, la cavité de réception débouchant par une ouverture proximale et par au moins une ouverture distale de prélèvement et/ou de réinjection ;
- un piston, monté mobile dans la cavité de réception pour obturer l'ouverture proximale.

Le fluide est avantageusement un fluide corporel tel qu'un tissu adipeux du derme et/ou de l'hypoderme d'un être humain ou animal. Le tissu est notamment destiné à jouer le rôle de réserve alimentaire et de couche protectrice contre le froid.

En variante, le fluide est un fluide biologique tel qu'un produit sanguin, un milieu de culture ou encore un élément figuré du sang.

Le dispositif du type précité est notamment destiné à être mis en oeuvre pour injecter un fluide dans un être vivant, par exemple pour effectuer un traitement cosmétique d'un être humain. Un tel traitement cosmétique est avantageusement une injection de fluide corporel sous la peau pour la retendre et faire diminuer ou disparaître des rides. En variante, le traitement cosmétique est une opération de reconstruction ou d'augmentation de volume d'une glande mammaire ou d'une masse charnue telle qu'une fesse.

Pour tendre la peau ou ré-augmenter son volume, il est connu de procéder à l'injection, sous la peau, d'un produit de remplissage, notamment au niveau du visage ou des glandes mammaires d'un être humain.

Ce type de traitement cosmétique est généralement réalisé à l'aide d'un produit artificiel ou naturel comme du collagène ou de l'acide galuronique.

L'utilisation d'un fluide extérieur au corps présente des désavantages. En particulier, les fluides injectés doivent être parfaitement stériles, et doivent être compatibles avec le corps humain pour éviter d'engendrer des infections, des réactions allergiques ou des rejets par l'organisme.

Pour pallier ce problème, il est connu de prélever du tissu adipeux dans les régions du corps présentant un excès de ce tissu, et d'utiliser le fluide corporel prélevé pour le réinjecter à un autre endroit dans le corps.

Le prélèvement de fluide dans le corps est réalisé par exemple à l'aide d'un dispositif de liposuccion qui comprend une aiguille, une tubulure et un réservoir recevant le tissu adipeux.

Cependant, avant de réinjecter le fluide corporel dans le patient, le chirurgien doit séparer le fluide adipeux en plusieurs phases pour éliminer au moins une partie de ce fluide. A cet effet, il prélève du fluide adipeux dans le réservoir, puis transfère ce fluide dans un récipient adapté pour une centrifugation.

Il sépare alors le fluide adipeux en plusieurs phases pour récupérer une phase propre à être réinjectée dans l'organisme. Le chirurgien doit donc supprimer le supernageant présent au dessus de la phase à réinjecter et ensuite prélever la phase à réinjecter à l'aide d'une seringue destinée à être piquée dans le corps du patient.

Compte tenu de la force d'injection nécessaire pour la réinjection, il est connu par ailleurs d'utiliser un injecteur mécanique propre à pousser le piston de la seringue avec une force suffisante pour provoquer l'injection du tissu adipeux.

Un tel procédé de traitement cosmétique ne donne pas satisfaction. En effet, un grand nombre de manipulations sont nécessaires pour prélever, centrifuger, et réinjecter le fluide corporel, ce qui est complexe à mettre en oeuvre, notamment dans l'environnement d'une salle d'opération.

En outre, compte tenu des transferts de fluide successifs entre le dispositif de prélèvement, le récipient destiné à être centrifugé, et la seringue de réinjection, l'asepsie de l'opération est très discutable et peut conduire à des risques d'infection.

Un but de l'invention est donc de fournir un dispositif de prélèvement et de réinjection d'un fluide corporel, notamment d'un tissu adipeux, le dispositif de prélèvement étant particulièrement simple et sûr à mettre en oeuvre.

A cet effet, l'invention a pour effet un dispositif du type précité, **caractérisé en ce que** le dispositif comporte un ensemble de distribution sélective d'une partie proximale du fluide située dans la cavité de réception, l'ensemble de distribution comportant un canal de circulation de fluide ménagé à travers le piston, le canal de circulation débouchant dans la cavité de réception.

Le dispositif selon l'invention peut comprendre l'une ou plusieurs des caractéristiques suivantes, prise(s) isolément ou suivant toutes combinaisons techniquement possibles :
- le piston comporte un corps distal d'obturation de la cavité et une tige d'actionnement du corps, le canal de circulation étant ménagé à travers le corps distal et à travers la tige ;
- l'ensemble de distribution comporte un piquage proximal de distribution raccordé au canal de circulation à l'opposé de la cavité de réception, le piquage proximal étant déplaçable conjointement avec le piston ;
- il comporte un raccord auxiliaire de mise sous vide de la cavité de réception, le raccord auxiliaire débouchant dans la cavité de réception à l'écart de l'ouverture distale ;
- il comporte une conduite de mise sous vide destinée à être raccordée d'une part, à la cavité de réception et d'autre part, à une source de vide.
- il comporte au moins une conduite amovible de prélèvement et/ou de réinjection destinée à être raccordée à la cavité de réception à travers l'ensemble de distribution ;
- l'extrémité proximale du corps creux est munie de moyens de verrouillage propres à être reçus dans des moyens de blocage complémentaire d'une centrifugeuse ;
- il comporte au moins une vessie recevant dans son volume intérieur au moins un premier embout raccordé à la cavité de réception et au moins un deuxième embout d'une conduite, le premier embout étant raccordable de manière réversible sur le deuxième embout.

L'invention a également pour objet un nécessaire de prélèvement et de réinjection d'un fluide, **caractérisé en ce qu'il** comporte
- un dispositif tel que décrit plus haut ;
- un injecteur comportant au moins un organe d'actionnement du piston, destiné à déplacer le piston dans la cavité de réception.

Le nécessaire selon l'invention peut comprendre l'une ou plusieurs des caractéristiques suivantes, prises isolément ou suivant toutes combinaisons techniquement possibles :
- il comporte une pompe à vide destinée à être raccordée hydrauliquement à la cavité de remplissage ;
- il comporte une centrifugeuse, la centrifugeuse comportant un berceau de réception du dispositif de prélèvement ;
- l'injecteur comprend :
   * un support de retenue du corps creux du dispositif ;
   * un organe d'actionnement monté mobile par rapport au support, l'organe d'actionnement étant destiné à coopérer avec le piston pour déplacer le piston dans la cavité de réception ;
- l'organe d'actionnement comporte un logement de réception de l'ensemble de distribution et/ou d'une conduite amovible destinée à être raccordée au canal de circulation de fluide.
- l'organe d'actionnement comporte une tige mobile axialement à travers le support, le logement débouchant axialement dans une surface d'appui sur le piston et débouchant transversalement à travers une surface latérale de la tige.

L'invention a aussi pour objet un injecteur destiné à recevoir un dispositif tel que décrit plus haut, l'injecteur comprenant :
- un support de retenue du corps creux du dispositif ;
- un organe d'actionnement monté mobile par rapport au support, l'organe d'actionnement étant destiné à coopérer avec le piston pour déplacer le piston dans la cavité de réception ;
   **caractérisé en ce que** l'organe d'actionnement comporte un logement de réception de l'ensemble de distribution et/ou d'une conduite amovible destinée à être raccordée au canal de circulation de fluide.

L'injecteur selon l'invention peut comprendre l'une ou plusieurs des caractéristiques suivantes, prises isolément ou suivant toutes combinaisons techniquement possibles :
- l'organe d'actionnement comporte une tige mobile axialement à travers le support, le logement débouchant axialement dans une surface d'appui sur le piston et débouchant transversalement à travers une surface latérale de la tige.

L'invention a aussi pour objet un procédé non thérapeutique de traitement cosmétique d'un être vivant, comprenant les étapes suivantes :
- fourniture d'un dispositif tel que décrit plus haut, la cavité de réception du corps contenant un fluide avantageusement non thérapeutique;
- évacuation sélective d'une partie proximale du fluide présent dans la cavité de réception, le piston obturant l'ouverture proximale, indépendamment d'une partie distale du fluide située entre la partie proximale et l'ouverture distale par l'intermédiaire de l'ensemble de distribution, la partie proximale du fluide étant évacuée à travers le canal d'évacuation ménagé dans le piston.

Le procédé selon l'invention peut comprendre la caractéristique suivante :
- il comporte une étape de séparation du fluide contenu dans la cavité de réception en une première phase proximale et en au moins une deuxième phase distale, le procédé comprenant les étapes suivantes :
- évacuation de la première phase proximale hors de la cavité de réception à travers l'ensemble de distribution sans évacuer la deuxième phase distale ;
- il comporte une étape de remplissage de la cavité par un fluide corporel.

L'invention sera mieux comprise à la lecture de la description qui va suivre, donnée uniquement à titre d'exemple, et faite en se référant aux dessins annexés, sur lesquels :
- la figure 1 est une vue schématique de côté d'un premier nécessaire de prélèvement et de réinjection de fluide corporel selon l'invention ;
- la figure 2 est une vue schématique, prise partiellement en coupe d'un dispositif de prélèvement et de réinjection avant son utilisation ;
- la figure 3 est une vue en élévation d'un détail marqué III sur la figure 1 ;
- la figure 4 est une vue analogue à la figure 3 en perspective de trois-quarts face ;
- la figure 5 est une vue analogue à la figure 2, lors d'une phase de prélèvement de fluide corporel dans un patient ;
- la figure 6 est une vue analogue à la figure 5, lors d'une phase d'élimination d'au moins une partie du fluide corporel ; et
- la figure 7 est une vue analogue à la figure 5 lors d'une phase de réinjection du fluide corporel dans un être humain.
- la Figure 8 illustre un deuxième nécessaire selon l'invention ;
- la Figure 9 illustre un troisième nécessaire selon l'invention ; et
- la Figure 10 illustre un quatrième nécessaire selon l'invention ;
- la Figure 11 est une vue analogue à la figure 7 pour un cinquième nécessaire selon l'invention ;
- la figure 12 est une vue d'un détail du nécessaire de la figure 11, illustrant un dispositif additionnel de réglage du débit.

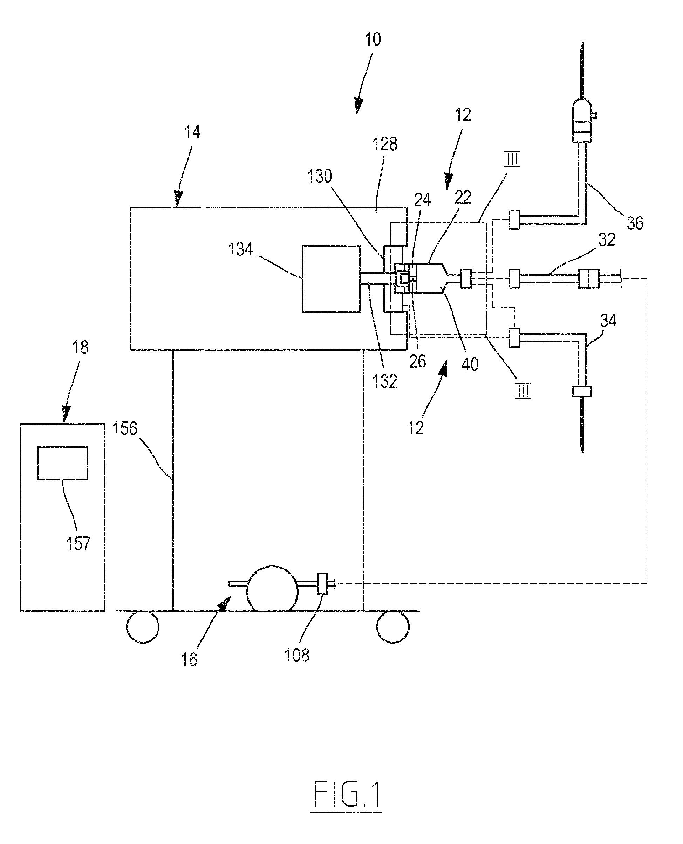

Un premier nécessaire 10 de prélèvement et de réinjection de fluide selon l'invention est illustré schématiquement par la figure 1.

Ce nécessaire 10 est avantageusement destiné à la mise en oeuvre d'un procédé de traitement cosmétique, non thérapeutique, sur un sujet formé par un être humain ou animal.

Le nécessaire 10 comporte un premier dispositif 12 de prélèvement et de réinjection, un injecteur mécanique 14, une pompe à vide 16 et une centrifugeuse 18

Le fluide corporel destiné à être prélevé et réinjecté par le dispositif 12 est par exemple un tissu adipeux du derme et/ou de l'hypoderme d'un être humain ou animal, notamment destiné à jouer le double rôle de réserve alimentaire et de couche protectrice contre le froid. Un tel tissu adipeux est généralement désigné par le terme « graisse dermique », « graisse de couverture », ou « graisse sous-cutanée ». Ce fluide, après prélèvement, est propre à être séparé en plusieurs phases, dont au moins une phase est compatible pour être réinjectée par le dispositif 12 selon l'invention.

En variante, le fluide est un fluide biologique tel qu'un produit sanguin, un milieu de culture ou encore un élément figuré du sang.

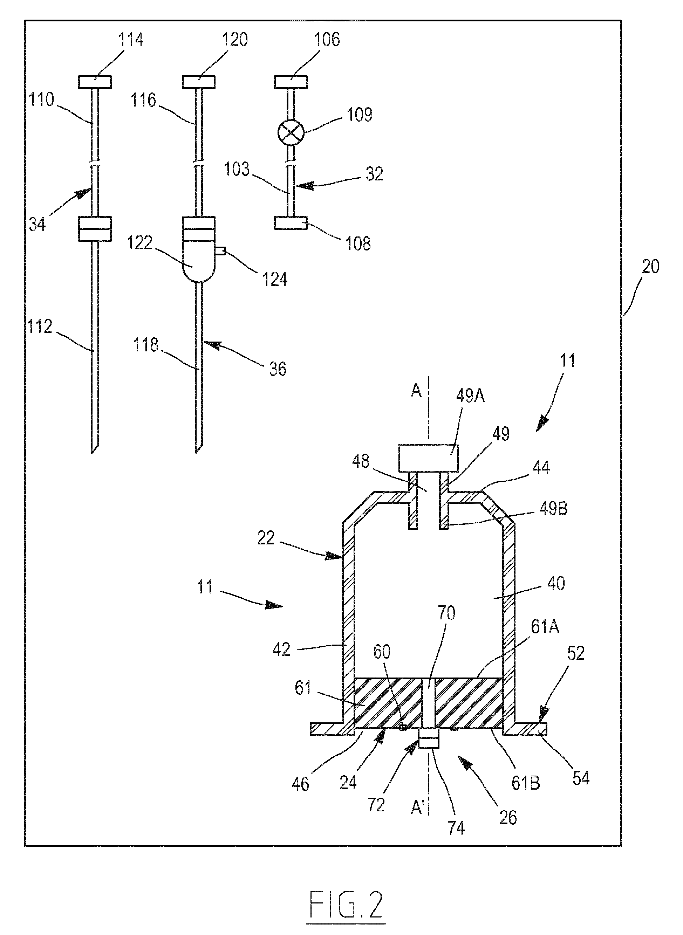

Comme illustré par la figure 2, le dispositif 12 est de préférence initialement contenu dans un emballage stérile 20.

Il comporte un ensemble 11 d'un seul tenant comportant un corps de seringue 22 creux, un piston 24 monté mobile dans le corps de seringue 22 et, selon l'invention, un ensemble de distribution 26 porté par le piston 24.

Dans l'exemple représenté sur la Figure 2, le dispositif 12 comporte en plus, une conduite amovible 32 de mise sous vide, une première conduite amovible 34 de prélèvement de fluide corporel et une deuxième conduite amovible 36 de réinjection de fluide corporel.

Le corps de seringue 22 est par exemple réalisé d'un seul tenant en un matériau transparent, tel que du verre ou une matière plastique. Il est destiné à être raccordé, dans une première étape de prélèvement, à la pompe à vide 16 et à la conduite de prélèvement 34 pour le prélèvement de fluide corporel dans le sujet.

Le corps 22 est ensuite destiné à être monté dans un berceau de la centrifugeuse 18 en vue de la séparation du fluide prélevé en plusieurs phases. Le corps 22 est par ailleurs destiné à être connecté à l'injecteur 14 et à la conduite de réinjection 36 pour permettre la réinjection d'au moins une phase du fluide corporel dans le sujet.

Le corps 22 présente une forme tubulaire s'étendant le long d'un axe A-A' représenté vertical sur la figure 2. Il délimite intérieurement une cavité 40 de réception du fluide corporel. Il comporte une paroi périphérique 42 et une paroi distale 44 obturant distalement la paroi périphérique 42.

La cavité 40 est délimitée extérieurement par la paroi périphérique 42 et distalement par la paroi distale 44. Elle débouche en amont par une ouverture proximale 46 principale ménagée à l'extrémité proximale de la paroi 42.

Elle débouche en aval par une ouverture 48 distale de prélèvement et de réinjection.

L'ouverture distale 48 est ménagée dans la paroi distale 44. Elle est située axialement à l'opposé de l'ouverture proximale 46. L'ouverture distale 48 présente une section transversale inférieure à la section transversale de l'ouverture principale 46.

Le corps 22 porte un raccord distal 49 qui fait saillie à partir de la paroi distale 44 autour de l'ouverture distale 48. Le raccord distal 49 est muni d'un embout 49A de raccordement destiné à être raccordé de manière amovible à une conduite 32, 34, 36. L'embout 49A est également propre à recevoir un organe d'obturation sélective de l'ouverture 48 tel qu'un bouchon ou une vanne.

Avantageusement, le raccord distal 49 comporte un tronçon proximal 49B faisant saillie dans la cavité 40 pour guider l'évacuation de fluide à travers l'ouverture 48.

Le volume de la cavité de réception 40 est par exemple compris entre 5 cm³ et 300 cm³.

Au voisinage de l'ouverture proximale 46, la paroi latérale 42 est munie extérieurement de moyens 52 de verrouillage du corps 22 sur l'injecteur 14 et sur un berceau de la centrifugeuse 18. Ces moyens 52 sont par exemple formés par un filetage, ou par une collerette de retenue 54 représentée schématiquement sur la figure 2.

Le piston 24 présente une section transversale extérieure conjuguée à la section transversale intérieure de la cavité 40. Il obture de manière étanche la cavité 40 dans une direction proximale.

Comme illustré par la Figure 2, le piston 24 est avantageusement muni de moyens 60 d'accrochage, destinés à être mis en prise avec un organe d'actionnement mobile présent dans l'injecteur 14.

Le piston 24 est monté mobile dans la cavité 40 le long de l'axe A-A', entre une position proximale, située au voisinage de l'ouverture proximale 46, et une position distale, située au voisinage de la paroi distale 44.

Lors de son déplacement le long de l'axe A-A', le piston 24 obture de manière étanche la cavité 40 à l'exception de l'ensemble de distribution 26. Ceci empêche le fluide présent dans la cavité 40 de s'échapper à travers l'ouverture proximale 46, sauf à passer par l'ensemble de distribution 26.

Comme illustré par la Figure 2, le piston 24 comporte un corps d'obturation 61, de section transversale extérieure conjuguée à la section transversale intérieure de la cavité 40.

Le piston 24 présente une surface interne 61 A disposée dans la cavité 40 et une surface externe opposée 61 B, dans laquelle sont ménagés les moyens d'accrochage 60 lorsqu'ils sont présents.

La surface externe 61 B est destinée à entrer en contact avec l'organe d'actionnement présent sur l'injecteur 14.

Selon l'invention, l'ensemble de distribution 26 comporte un canal 70 de circulation de fluide ménagé à travers le piston 24, et avantageusement, un piquage proximal 72 de raccordement.

Dans l'exemple représenté sur la figure 2, le canal de circulation 70 est ménagé axialement à travers le corps 61 du piston 24. Il débouche dans la surface interne 61 A et dans la surface externe 61 B. Il raccorde fluidiquement la cavité 40 au piquage proximal 72.

Le piquage proximal 72 fait saillie extérieurement à partir de la surface externe 61 B. Il est raccordé au canal de circulation 70. Il est monté mobile conjointement avec le piston 24 lors du déplacement du piston 24.

Le piquage proximal 72 est muni d'un embout proximal 74 destiné à être raccordé sélectivement à une conduite 34, 36, ou à un obturateur constitué par exemple par un bouchon ou une vanne.

Dans cet exemple, le canal 70 est sensiblement parallèle à l'axe A-A', co-axialement avec l'axe A-A'. En variante, le canal 70 est décalé transversalement par rapport à l'axe A-A' ou/et s'étend de manière inclinée par rapport à l'axe A-A'.

La conduite de mise sous vide 32 est par exemple réalisée à base d'une tubulure rigide 103. Elle comporte un embout amont 106 destiné à être monté sur l'embout distal 49A du raccord distal 49 et un embout aval 108 destiné à être connecté à la pompe à vide 16. La conduite de mise sous vide 32 est avantageusement munie d'une vanne d'obturation 109.

Comme illustré par la figure 2, la conduite de prélèvement 34 comporte une tubulure de liaison 110, avantageusement flexible, et une aiguille 112 de prélèvement plus rigide que la tubulure 110. L'aiguille 112 est constituée par exemple par une aiguille de liposuccion. La tubulure 110 présente un embout amont 114 destiné à être raccordé à l'embout 74 du piquage proximal 72 ou/et à l'embout 49A du raccord distal 49.

La conduite 36 de réinjection comprend une tubulure flexible 116 de réinjection munie en aval d'une aiguille de réinjection 118, plus rigide que la tubulure 116. Sur la

Figure 2, la conduite de réinjection 36 est avantageusement distincte de la conduite de prélèvement 34. En variante, la conduite 34 forme à la fois la conduite de prélèvement et la conduite de réinjection.

La tubulure de réinjection 116 est munie d'un embout 120 destiné à être raccordé à l'embout 49A du raccord distal 49 ou/et à l'embout 74 du piquage proximal 72.

Dans une variante avantageuse, un module 122 de commande du débit de fluide corporel réinjecté est disposé sur la conduite de réinjection 36. Ce module 122 comporte un organe de commande 124 pilotable par un opérateur pour contrôler le débit circulant à travers la tubulure 116 et à travers l'aiguille 118 de réinjection.

En variante, le module de commande 122 est disposé en amont de la conduite de réinjection 36, entre le raccord distal 49 et l'embout 114, l'organe de commande 124 pouvant être déporté par rapport au module 122.

L'organe de commande 124 est par exemple, un bouton, comme représenté sur la figure 2. En variante, l'organe de commande 124 est une pédale, dans le cas où l'opérateur doit disposer de ses deux mains.

Chacune des conduites 32, 34, 36 est destinée à être montée de manière amovible et réversible respectivement sur l'un ou/et l'autre du raccord distal 49 et du piquage proximal 72.

En référence aux figures 1, 2 et 3, l'injecteur 14 selon l'invention comporte un bâti 128, un support 130 de réception du corps de seringue 22, un organe 132 d'actionnement du piston 24 et un berceau 133 de réception de l'organe d'actionnement 132.

L'injecteur 14 comprend en outre des moyens de commande 134 du déplacement de l'organe d'actionnement 132.

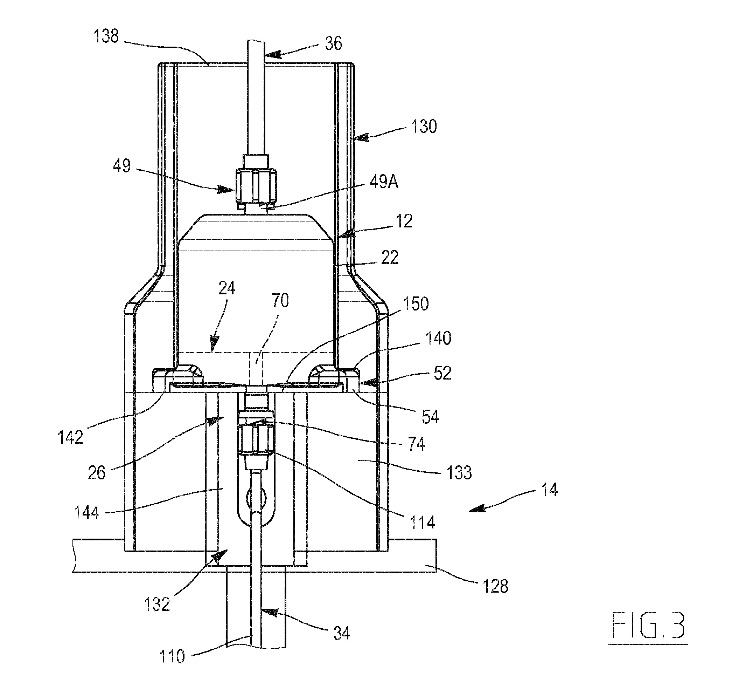

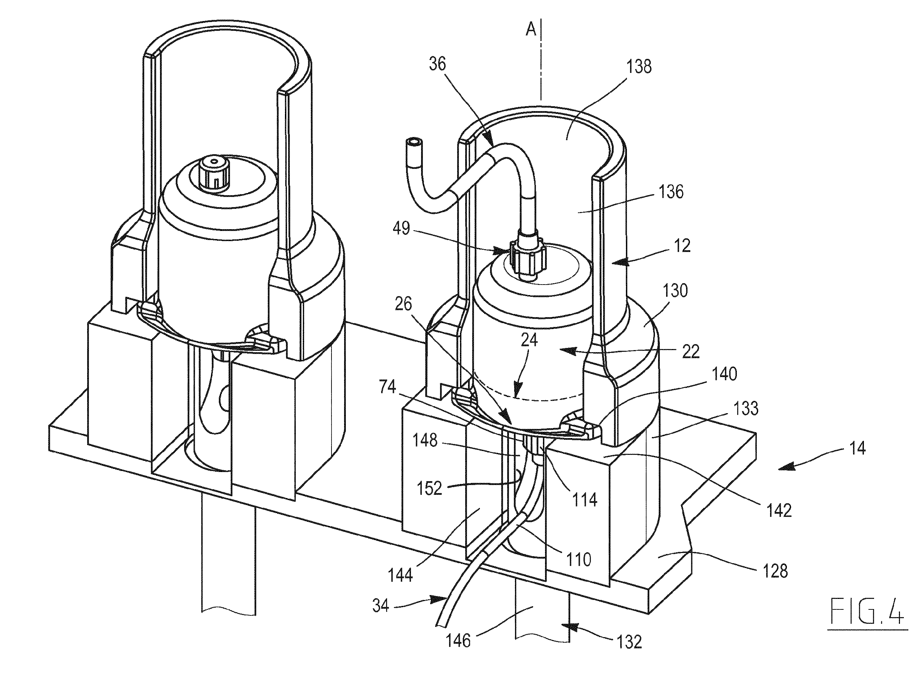

Comme illustré par les Figures 3 et 4, le support 130 est par exemple formé par un demi-fût monté sur le berceau 133. Le support 130 présente ainsi avantageusement une ouverture latérale 136 d'introduction du corps 22 et une ouverture supérieure 138 de passage d'une conduite 34, 36.

Le support 130 présente des moyens de blocage complémentaires aux moyens de verrouillage 52 du corps 22 disposés au voisinage de l'ouverture proximale 46.

Les moyens de blocage complémentaires sont ménagés dans le support 130. Ils sont par exemple formés par des fentes 140 définissant un épaulement de retenue des moyens de verrouillage 52.

Le corps 40 peut ainsi être introduit dans le support 130 par l'intermédiaire de l'ouverture latérale 136 en engageant de manière amovible les moyens de verrouillage 52 dans les fentes 140.

Le berceau de réception 133 de l'organe d'actionnement est disposé entre le bâti 128 et le support 130. Il délimite une surface supérieure 142 d'appui du corps 40 et une lumière centrale 144 de passage de l'organe d'actionnement 130.

La lumière 144 débouche en regard du support 130. Elle débouche par un passage latéral situé sous l'ouverture latérale 136 pour permettre le passage du piquage proximal 72 et éventuellement, d'une conduite 32, 34, 36 lors de l'insertion du corps 22 dans le support 130.

L'organe d'actionnement 132 comporte une tige 146 délimitant un logement 148. Le logement 148 est destiné à recevoir le piquage proximal 72 et éventuellement une conduite 32, 34, 36.

La tige 146 s'étend à travers le bâti 128, à travers le berceau 133, et à travers le support 130 lorsqu'elle est déployée par rapport au bâti 128. Elle présente une surface supérieure 150 d'appui sur le piston 24. La surface supérieure 150 est située à son extrémité libre.

La surface supérieure 150 est destinée à coopérer avec les moyens d'accrochage 60 pour déplacer le piston 24 dans la cavité 40.

Dans cet exemple, le logement 148 est formé par une échancrure axiale.

Plus généralement, le logement 148 débouche axialement suivant l'axe de la tige 146 dans la surface supérieure 150 par une ouverture axiale destinée à s'étendre en regard de la surface externe 61 B du piston 24.

Le logement 148 débouche transversalement par une ouverture latérale 152 permettant le passage d'une conduite 34, 36 et l'insertion du piquage proximal 72. L'ouverture latérale 152 s'étend en regard du passage latéral par lequel débouche la lumière 144.

L'organe d'actionnement 132 est ainsi mobile le long d'un axe A-A' dans le berceau 133 et dans le support 130 entre une position rétractée dans le berceau 133 et une position déployée dans le support 130, par l'intermédiaire des moyens de commande 134.

Lors de son déplacement, l'organe d'actionnement 132 déplace le piston 24 de sa position proximale à sa position distale.

L'organe d'actionnement 132 déplace conjointement le piquage proximal 72 et éventuellement la conduite 32, 34, 36 montée sur l'embout 74 du piquage 72.

Ainsi, le corps de seringue 22 peut être monté de manière réversible dans le support 130 sur l'injecteur 14. Une fois monté sur l'injecteur 14, l'organe d'actionnement 132 est apte à coopérer avec les moyens d'engagement 60 sur le piston 24 pour déplacer le piston 24 depuis sa position proximale vers sa position distale, à une vitesse contrôlée. La pompe à vide 16 est propre à engendrer un vide, par exemple de l'ordre de -950 mbars relatif dans la cavité de réception 40, lorsque la conduite de mise sous vide 32 est raccordée à la fois à la pompe à vide 16 et au raccord distal 49, et lorsque la vanne 109 est ouverte.

Dans une variante avantageuse, représentée sur la figure 1, l'injecteur 14 et la pompe à vide 16 sont reçus sur le même châssis 156 pour être déplaçables conjointement.

La centrifugeuse 18 comporte au moins un berceau 157 (visible sur la Figure 1) propre à recevoir de manière fixe le corps de seringue 22, l'ensemble de prélèvement 26 et le raccord distal 49.

Le berceau 157 présente un logement de réception (non représenté) destiné à recevoir l'extrémité proximale du corps de seringue 22 et les moyens de verrouillage 52.

Le logement du berceau 157 est muni de moyens de blocage complémentaires aux moyens de verrouillage 52. Il comporte en outre une butée de blocage en position du piston 24 pour empêcher son déplacement à travers l'ouverture proximale 46.

Une fois reçu dans le berceau 157, le corps de seringue 22 est verrouillé en position de manière réversible et est apte à être entraîné en rotation pour provoquer une séparation de phases au sein du fluide contenu dans la cavité de réception 40.

Le fonctionnement du nécessaire de prélèvement 10 selon l'invention, lors d'une opération non thérapeutique de traitement cosmétique d'un être vivant, va maintenant être décrit.

Initialement, en référence à la Figure 2, un dispositif 12 de prélèvement et de réinjection, avantageusement contenu dans un emballage stérile 20, est fourni.

L'opérateur ouvre alors l'emballage 20 et extrait le corps 22 de seringue, le piston 24 et l'ensemble de prélèvement 26.

Puis, il connecte la conduite de mise sous vide 32 au raccord distal 49 et ouvre la vanne 109 pour libérer la tubulure 103. Par ailleurs, il raccorde la conduite de prélèvement 34 au piquage proximal 72.

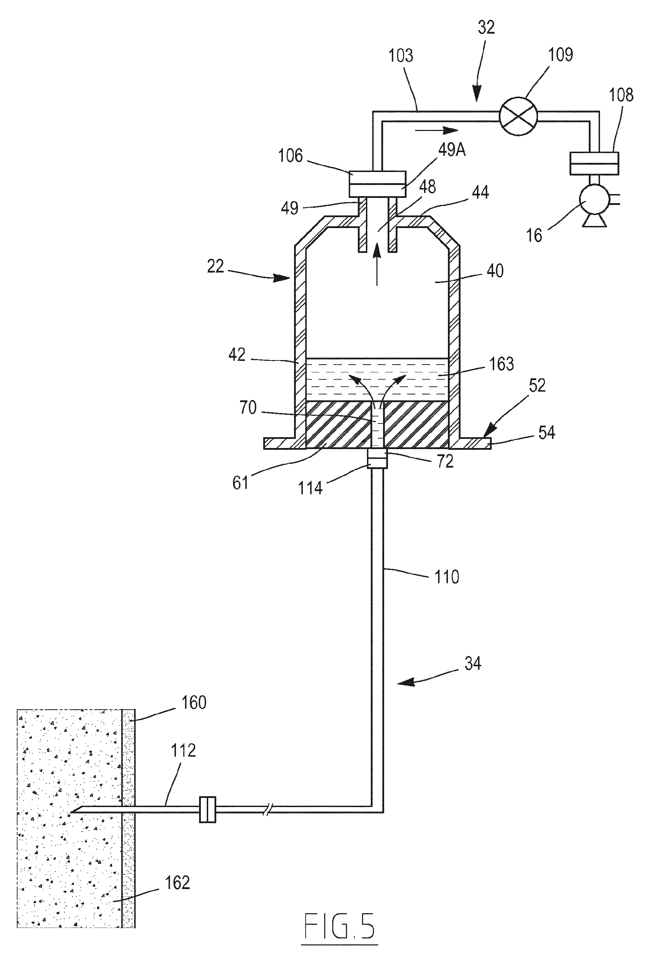

Une fois raccordée (Figure 5), la conduite de prélèvement 34 est saisie par l'opérateur. Celui-ci pique alors la peau 160 du sujet subissant le traitement cosmétique à l'aide de l'aiguille 112, pour prélever du tissu adipeux 162 sous la peau 160.

L'opérateur active ensuite la pompe à vide 16, ce qui provoque l'établissement d'une dépression dans la cavité 40 via la conduite de mise sous vide 32 et l'ouverture distale 48.

Le piston 24 occupe initialement sa position proximale au voisinage de l'ouverture proximale 46.

Sous l'effet de la dépression, du fluide 163 est aspiré successivement à travers l'aiguille de prélèvement 112, la tubulure de prélèvement 110, le piquage proximal 72 et le canal de circulation 70, afin de remplir progressivement la cavité 40, de bas en haut, comme illustré par la figure 5.

Une fois la cavité 40 au moins partiellement remplie, l'opérateur démonte la conduite de prélèvement 34 et la conduite de mise sous vide 32. Il bouche le raccord distal 49 et le piquage proximal 72, par exemple au moyen d'un bouchon ou d'une vanne (non représentée).

Puis, l'opérateur saisit le corps de seringue 22 obturé en aval par le piston 24 et introduit l'extrémité proximale du corps 22 dans un berceau 157 de la centrifugeuse 18.

L'opérateur engage ensuite les moyens de verrouillage 52 dans le logement pour bloquer en position le corps 22, et pour bloquer le piston 24 dans le berceau 157. Le piston 24 est alors immobilisé axialement dans le berceau 157.

Puis, l'opérateur active la centrifugeuse 18 pour séparer le fluide 163 contenu dans la cavité de réception 40 en plusieurs phases 164A, 164B, 164C représentées sur la

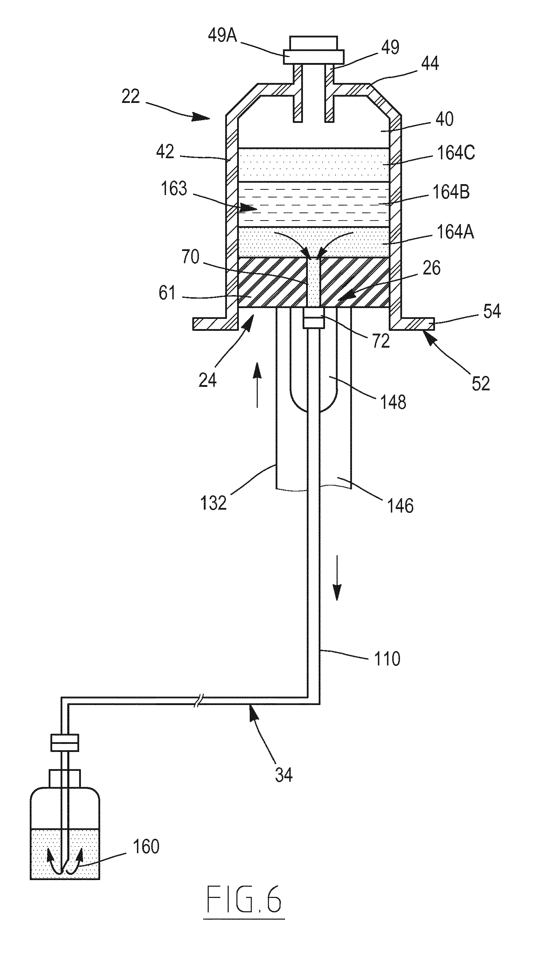

Figure 6. Cette séparation est obtenue par mise en rotation du berceau 157 contenant le corps 22.

Une fois l'opération de séparation effectuée, l'opérateur démonte le corps de seringue 22 pour l'extraire hors de la centrifugeuse 18 en libérant les moyens de verrouillage. Ensuite, comme illustré par la figure 5, l'opérateur raccorde une conduite d'évacuation, par exemple formée par la conduite de prélèvement 34, sur le piquage proximal 72.

Puis, il dispose le corps 22 verticalement sur l'injecteur 14 pour éliminer les phases 164A, 164C respectivement inférieure et supérieure, en ne conservant qu'une phase intermédiaire 164B pour la réinjection dans le sujet.

A cet effet, il introduit le corps 22 dans le support 130. En référence aux Figures 3 et 4, le piquage proximal 72 et une partie de la conduite 34 pénètrent transversalement dans le logement 148 ménagé à l'extrémité libre de la tige 146 par l'intermédiaire de l'ouverture latérale 152 et de la lumière 144.

Comme illustré par la Figure 4, l'embout amont 114 de la tubulure 110, le piquage proximal 72 et la partie amont de la tubulure 110 sont alors reçus dans le logement 148. Une partie de la tubulure 110 fait saillie hors du logement 148 à travers la lumière 144, et, hors du berceau 133.

Lors de cette opération, le raccord distal 49 reste obturé.

Ensuite, comme illustré par la figure 6, l'opérateur active les moyens de commande 134 de l'injecteur 14 pour déplacer le piston 24 le long de l'axe A-A' vers l'extrémité distale du corps 22.

Lors de ce déplacement, la phase inférieure 164A est évacuée successivement à travers le canal 70, le piquage 72, puis à travers la conduite d'évacuation jusqu'à un conteneur 160, en vue d'être éliminée.

Puis, une fois la phase inférieure 164A éliminée, l'opérateur obture le piquage proximal 72. Il monte une conduite d'évacuation, par exemple la conduite de prélèvement 34, sur le raccord distal 49 du corps 22.

Puis, l'opérateur actionne à nouveau les moyens de commande 134 pour déplacer le piston 24 vers l'extrémité distale du corps 22 et évacuer ainsi la phase supérieure 164C à travers le raccord distal 49, puis à travers la conduite d'évacuation 34.

Ceci étant fait, l'opérateur démonte la conduite d'évacuation et monte la conduite de réinjection 36 sur le raccord distal 49. La surface supérieure de la phase intermédiaire 164B est alors située au niveau du tronçon proximal 49B du raccord distal 49.

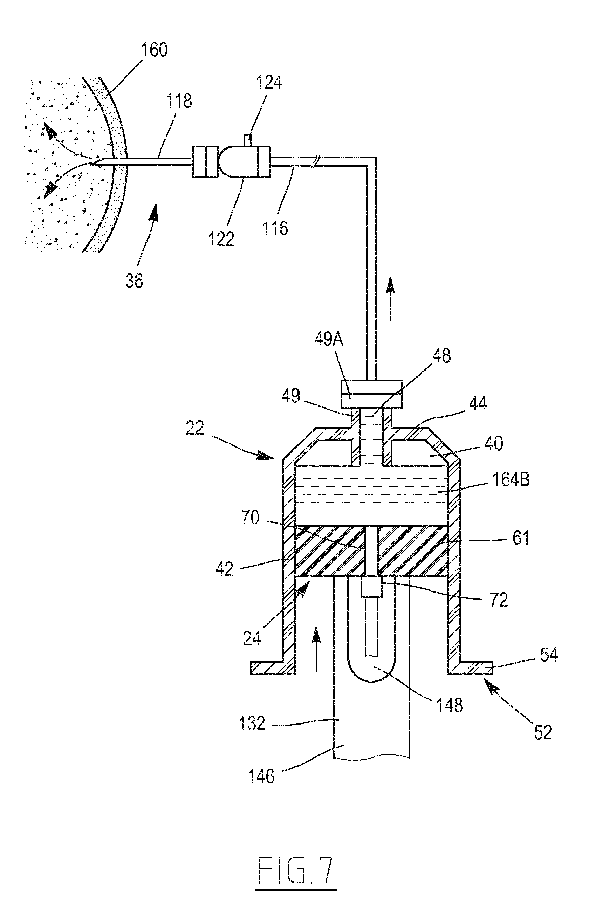

Puis, en référence à la Figure 7, l'opérateur pique la peau 160 du sujet dans une région distincte de celle où le tissu adipeux 162 a été prélevé, par exemple au niveau du visage, dans les glandes mammaires ou dans une partie charnue.

L'opérateur actionne alors l'organe de commande 124, ce qui active les moyens de commande 134 et le déplacement de l'organe d'actionnement 132, pour pousser le piston 24 vers l'extrémité distale du corps 22. Ceci provoque l'évacuation de la phase sélectionnée 164B du fluide corporel présente dans la cavité 40 à travers successivement le raccord distal 49, la tubulure de réinjection 116, le raccord de commande 122 et l'aiguille de réinjection 118.

Le dispositif 12 selon l'invention permet donc de réaliser successivement toutes les opérations nécessaires au prélèvement, à la centrifugation, et à la réinjection du fluide, sans avoir à changer le fluide de récipient. L'asepsie de l'opération s'en trouve renforcée et le risque de contamination microbienne est très faible.

De plus, le dispositif 12 est extrêmement simple à manier, notamment dans l'environnement d'une salle d'opération. En particulier, la présence de l'ensemble de distribution 26 solidaire du piston 24 permet d'éliminer toutes les phases du fluide qui ne sont pas désirées pour injecter uniquement une ou plusieurs phases sélectionnée(s).

Le dispositif 12 permet donc une manipulation propre et simple du fluide corporel, de manière, sans risque de fuite ou de contamination.

Plus généralement, le fluide corporel prélevé dans un premier être vivant n'est pas nécessairement réinjecté dans ce premier être vivant, mais est réinjecté dans un deuxième être vivant.

Il résulte donc de ce qui précède que l'ensemble de distribution 26 est propre à évacuer sélectivement une partie amont du fluide présent dans la cavité de réception 40, indépendamment d'une partie aval du fluide située entre la partie amont et la ou chaque ouverture distale 48.

La partie amont du fluide est avantageusement constituée par la phase inférieure 164A qui peut être évacuée par l'intermédiaire de l'ensemble de distribution 26, indépendamment de la phase intermédiaire 164B, et sans avoir à extraire la phase intermédiaire 164B par une ouverture distale 48.

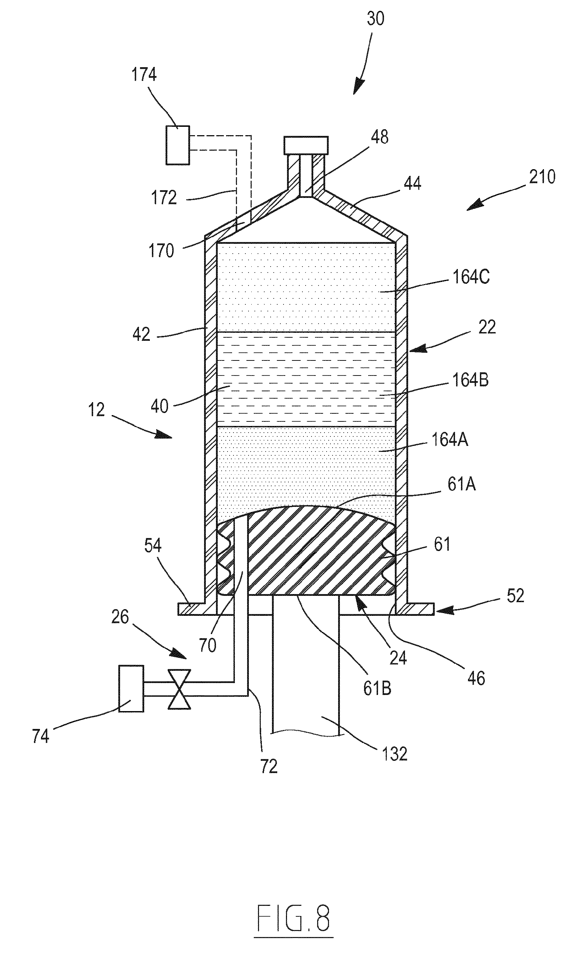

Un deuxième nécessaire 210 selon l'invention est illustré par la Figure 8. Ce nécessaire 210 est analogue au premier nécessaire 10.

Comme illustré par la Figure 8, le dispositif 12 de prélèvement et de réinjection du deuxième nécessaire 210 diffère du dispositif 12 du premier nécessaire 10 par la caractéristique selon laquelle l'ensemble de distribution 26 est décalé axialement par rapport à l'axe du piston 24.

Le fonctionnement du deuxième nécessaire 210 est analogue à celui du premier nécessaire 10.

Le dispositif 12 peut alors être monté sur un injecteur 14 dont l'organe d'actionnement 132 n'est pas nécessairement muni d'un logement 148 de réception du piquage proximal 72.

Le piquage proximal 72 fait saillie à l'écart de l'organe d'actionnement 132 par rapport au piston 24.

Dans une variante du dispositif 12, représentée en pointillés sur la Figure 8, le corps 22 comporte une ouverture distale additionnelle 170 distincte de l'ouverture distale 48. L'ouverture distale additionnelle 170 débouche dans un raccord auxiliaire 172 monté sur le corps 22 et muni d'un embout 174 de connexion à une conduite 32, 34, 36.

Ainsi, l'ouverture auxiliaire 170 peut être raccordée à la conduite de mise sous vide 32, et l'ouverture principale 48 peut être raccordée à la conduite de prélèvement 34 et/ou la conduite de réinjection 36. Cette variante s'applique également au premier nécessaire 10.

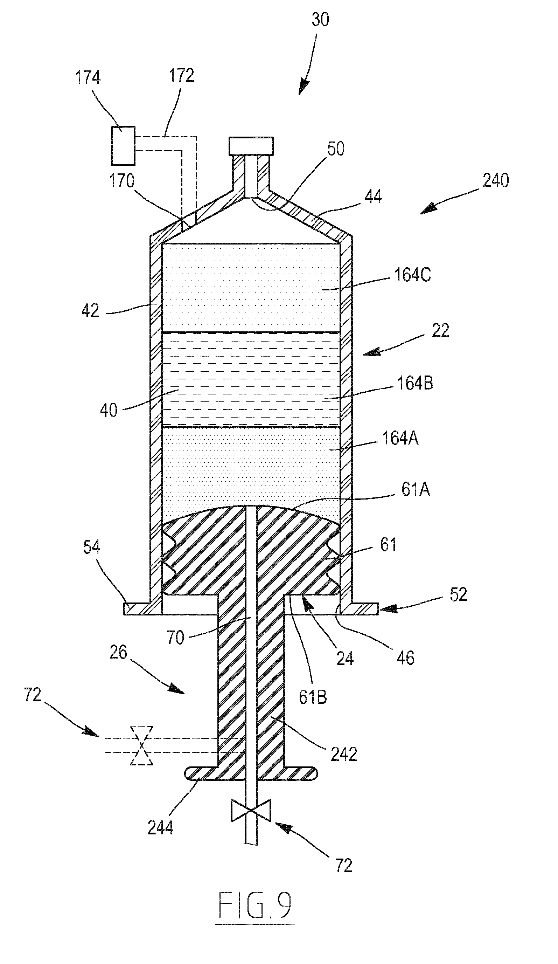

Un troisième nécessaire selon 240 selon l'invention est illustré par la Figure 9.

A la différence du deuxième nécessaire 210, le piston 24 comporte, outre le corps d'obturation 61, une tige proximale 242 d'actionnement du piston 24 qui fait saillie extérieurement à partir du corps d'obturation 61. Dans cet exemple, le canal 70 s'étend successivement à travers le corps 61, puis à travers la tige 242, pour déboucher hors de la tige 242, avantageusement au niveau de l'extrémité proximale 244 de la tige 242. Le piquage proximal 72 est raccordé sur la tige 242.

Dans le dispositif représenté sur la Figure 9, le raccord proximal 72 fait saillie axialement, dans l'axe de la tige 242 du piston.

Dans une variante représentée en pointillés, le raccord proximal 72 fait saillie transversalement par rapport à l'axe de la tige 242. Le canal 70 est alors coudé.

Le fonctionnement du troisième nécessaire 240 est par ailleurs analogue à celui du premier nécessaire 10.

Dans une variante, le piston 24 n'est pas actionné par un injecteur 14. Il est actionné manuellement par un opérateur à l'aide de ses doigts.

Ceci s'applique notamment pour les petites seringues de volume inférieur à 50 ml et notamment compris entre 5 ml et 15 ml ou des tubes de prélèvement de même volume pour évacuer un sous nageant sans contaminer la ou les phases à conserver.

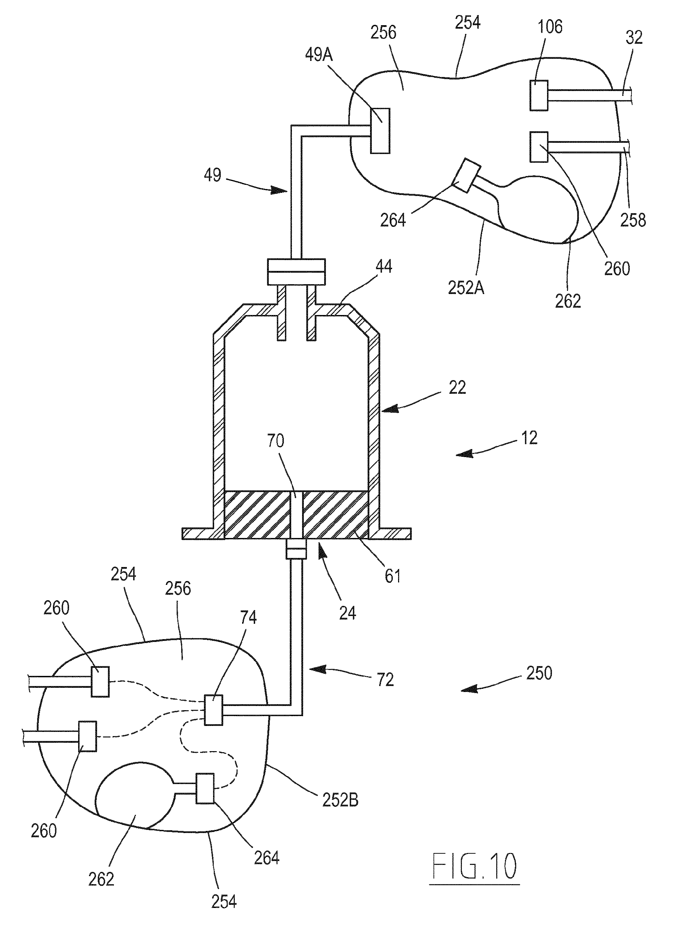

Un quatrième nécessaire 250 selon l'invention est illustré par la Figure 10. A la différence du dispositif 12 du premier nécessaire 10, le dispositif 12 du quatrième nécessaire 250 comporte au moins une vessie de protection 252A, 252B destinée à assurer l'asepsie lors de la connexion des conduites 32, 34, 36 respectivement sur l'un et/ou l'autre du raccord distal 49 et du piquage proximal 72.

Le dispositif 12 représenté sur la Figure 10 comporte avantageusement une première vessie 252A montée sur le raccord distal 49 et une deuxième vessie 252B montée sur le piquage proximal 72.

Chaque vessie 252A, 252B est formée par une enveloppe souple 254, avantageusement transparente. L'enveloppe souple 254 délimite un volume intérieur 256 gazeux sensiblement étanche.

L'enveloppe 254 de la première vessie 252A est montée de manière étanche autour d'une partie distale du raccord distal 49. Elle reçoit l'embout 49A du raccord distal 49 dans son volume intérieur 256.

La première vessie 252A est également montée de manière étanche autour de la partie proximale de la conduite de mise sous vide 32. Elle est avantageusement montée de manière étanche autour d'une extrémité proximale d'une conduite d'évacuation 258.

Le volume 256 reçoit ainsi avantageusement l'embout amont 106 de la conduite de mise sous vide et l'embout amont 260 de la conduite d'évacuation 258.

Dans une variante avantageuse, la vessie 252A contient en outre au moins une poche 262 de récupération d'une phase à éliminer, munie d'un embout 264 à son extrémité.

Ainsi, l'embout 49A du raccord distal 49 peut être raccordé sélectivement à l'un des embouts 106, 260, 264 contenus dans la vessie 252A lors des différentes phases du procédé. Cette opération peut être effectuée sans que l'opérateur ait à toucher les embouts 106, 260, 264, par simple déformation de l'enveloppe 254.

De même, l'enveloppe 254 de la deuxième vessie 252B contient l'embout 74 du piquage proximal. Cet embout 74 peut être raccordé sélectivement à un embout 260 d'une conduite d'évacuation 258 ou à un embout 264 d'une poche 262, les embouts 74, 260, 264 étant reçus dans l'enveloppe 254 de la deuxième vessie 252B.

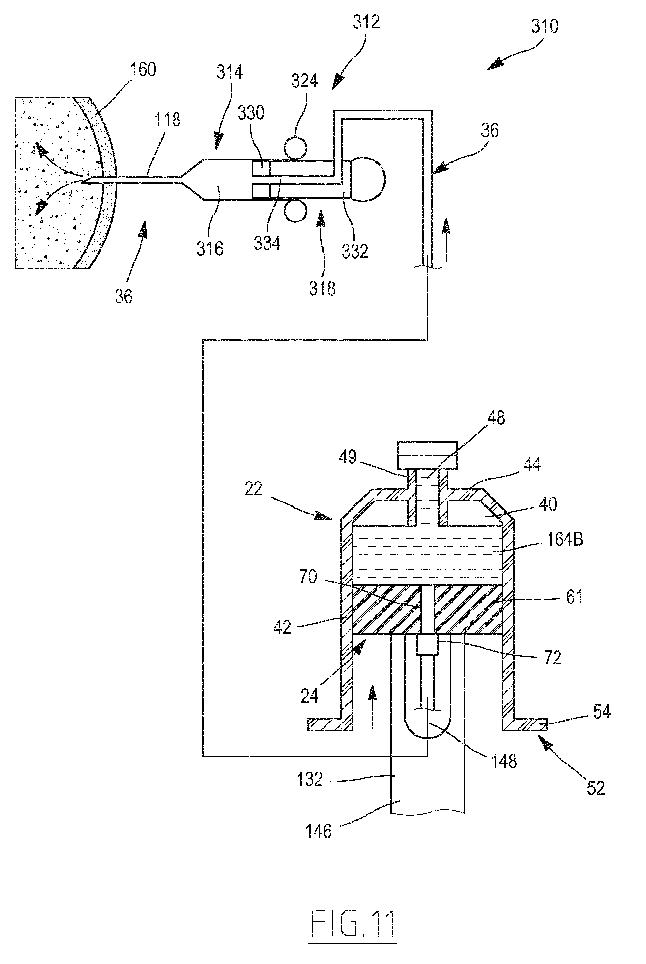

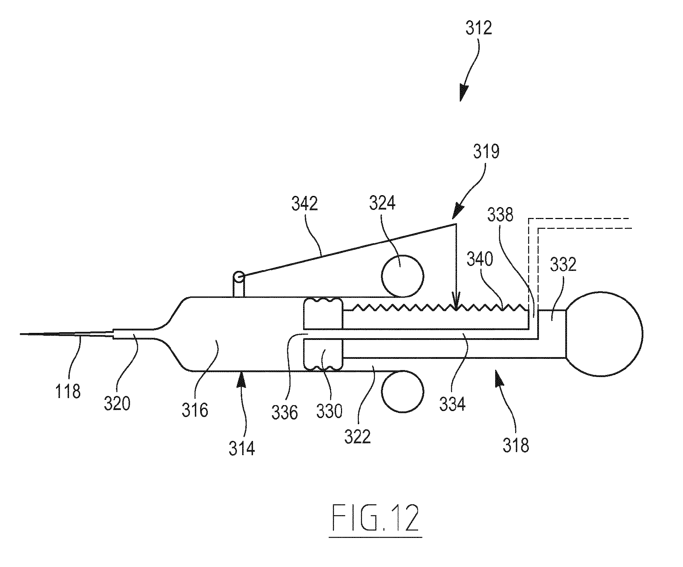

Un cinquième nécessaire 310 selon l'invention est illustré par les figures 11 et 12.

Comme le premier nécessaire 10, le cinquième nécessaire 310 comporte un premier dispositif 12 de prélèvement et de réinjection, destiné à être monté de manière amovible sur un injecteur mécanique 14.

Le cinquième nécessaire 310 selon l'invention diffère du premier nécessaire 10 en ce qu'il comporte un dispositif additionnel 312 de réglage manuel du débit de fluide réinjecté dans le sujet.

Le dispositif 312 est raccordé à l'organe de réinjection 118, qui est avantageusement une aiguille, ou une canule fine.

Dans cet exemple, le dispositif additionnel 312 est formé par une seringue qui porte l'organe de réinjection 118.

Comme illustré par la figure 12, le dispositif 312 comporte un corps creux additionnel 314 délimitant un volume intérieur 316 de régulation de débit. Le dispositif 312 comporte en outre un piston 318 mobile dans le corps 316 pour obturer en amont le volume intérieur 316.

Dans cet exemple, le dispositif 312 comporte en outre avantageusement un mécanisme libérable 319 de blocage en position du piston 318 par rapport au corps 314.

Le corps creux additionnel 314 présente une forme tubulaire. Il délimite une ouverture distale 320 raccordée à l'aiguille 118, et une ouverture proximale 322 à travers laquelle le piston 318 est inséré dans le corps 314.

Le corps 314 comporte, autour de son ouverture proximale 322, une collerette 324 propre à être saisie par les doigts d'un utilisateur pour maintenir en position le corps creux 314.

Le piston 318 comporte une tête 330 d'obturation du volume 316, insérée dans le corps 114 et une tige 332 d'actionnement, propre à faire saillie hors du corps 314 pour être poussée par un doigt d'un utilisateur.

Le piston 318 délimite intérieurement un canal 334 d'amenée de fluide dans le volume 316. Le canal 334 débouche en aval par un orifice aval 336 situé dans le volume 316, et en amont, par un orifice amont 338 situé hors du corps 314.

L'orifice amont 338 est destiné à être raccordé à la conduite 36 de réinjection de fluide. Dans l'exemple représenté sur la figure 12, il débouche transversalement par rapport un axe du piston 318.

Dans cet exemple, le mécanisme 319 comporte une crémaillère de blocage 340 présente sur le piston 318, et un organe mobile 342 de mise en prise de la crémaillère 340, monté sur le corps 314.

L'organe de mise en prise 342 est mobile entre une position de libération du piston 318 dans laquelle il est situé à l'écart de la crémaillère 340, et une pluralité de positions de blocage du piston 318 par rapport au corps 314, dans laquelle il est en prise avec au moins une dent de la crémaillère 340.

Dans l'exemple représenté sur la figure 11, la conduite de réinjection 36 est raccordée en amont au canal de circulation 70 ménagé dans le piston 24. Elle est logée au moins partiellement dans le logement 148 ménagé dans la tige 146 de l'organe d'actionnement 132.

Le fonctionnement du cinquième nécessaire 310 selon l'invention est analogue à celui du premier nécessaire 10 jusqu'à la fin de l'étape de séparation et d'élimination des phases 164A, 164C non désirées pour la réinjection dans le sujet.

Le fonctionnement du cinquième nécessaire 310 selon l'invention diffère du fonctionnement du premier nécessaire 10 lors de l'étape de réinjection.

Avant cette étape, l'opérateur raccorde la conduit de réinjection 36 au corps 22 du dispositif 12. Dans cet exemple, il monte l'extrémité amont de la conduite 36 sur le raccord 72 pour relier le canal 70 à la conduite 36. Par ailleurs, il fixe l'extrémité aval de la conduite de réinjection 36 sur le piston 318 du dispositif additionnel 312 pour raccorder la conduit de réinjection 36 au canal 334. Il bouche par ailleurs le raccord distal 49.

Dans un premier temps, l'opérateur procède au remplissage du volume intérieur 316 du dispositif additionnel 312. À cet effet, l'opérateur active l'injecteur 14, ce qui provoque un déplacement de la tige 146 de l'organe d'actionnement 132 vers l'intérieur du volume 40 pour pousser le piston 24.

Ce déplacement du piston 24 engendre l'évacuation de fluide 164B à travers la conduite 36, et à travers le canal 334 jusqu'au volume intérieur 316.

Lorsque le volume intérieur 316 est rempli, l'opérateur pique la peau 160 du sujet.

Dans un premier mode d'opération, lorsqu'une injection de fluide à débit constant est requise, l'opérateur règle la vitesse de déplacement constante de l'organe d'actionnement 132, et maintient en position le piston 318 du dispositif additionnel 312 par rapport au corps 314.

Ce maintien en position est assuré soit manuellement, soit à l'aide du mécanisme 319. Le volume intérieur 316 reste constant.

Dans ce mode d'opération, un débit constant de fluide est injecté dans le sujet, de manière automatique, par l'injecteur 14.

Dans un deuxième mode d'opération, lorsque le débit de fluide injecté doit être ponctuellement augmenté, l'opérateur déplace manuellement le piston 318 du dispositif additionnel 312 vers l'ouverture aval 320 pour réduire le volume 316. Ce déplacement du piston 318 est effectué sans modifier le réglage de débit de l'injecteur 14.

Un débit supérieur de fluide est alors éjecté ponctuellement hors du dispositif additionnel 312, ce qui permet par exemple d'effectuer un remplissage rapide d'une cavité ou d'une zone corporelle pour augmenter son volume.

Dans un troisième mode d'opération, lorsque le débit de fluide injecté doit être ponctuellement diminué, l'opérateur libère le piston 318 du dispositif additionnel 312 et le laisse libre de se déplacer à l'écart de l'ouverture aval 320. Ceci provoque l'augmentation du volume intermédiaire 316 et la diminution correspondante du débit de fluide extrait à travers l'aiguille 118. En variante, l'opérateur déplace manuellement le piston 318 à l'écart de l'ouverture aval 320.

Un tel mode d'opération permet de contrôler précisément la quantité de fluide extrait hors du dispositif additionnel 312, notamment pour une injection précise de fluide additionnel dans le sujet.

La présence d'un dispositif additionnel 312 permet donc un réglage précis de la quantité de fluide extraite hors du nécessaire 310, en vue de sa réinjection dans un sujet. Ce réglage est effectuée manuellement par un simple actionnement du piston 318 à l'aide des doigts de l'opérateur, sans avoir à agir sur le réglage de l'injecteur 14.

Ainsi, dans des conditions normales d'opération, l'opérateur maintient en position le piston 318. La quantité de fluide injectée est alors contrôlée directement par l'injecteur 14. L'opérateur n'effectue aucun effort.

Lorsqu'un complément de fluide doit être ajouté plus rapidement et ponctuellement, une simple manipulation manuelle du piston 318 permet de contrôler précisément la quantité de fluide injectée sans avoir à modifier le réglage de l'injecteur 14. Ceci est également le cas lorsque le débit de fluide éjecté hors du nécessaire 310 doit être ponctuellement réduit, notamment dans une zone où l'injection doit être très précise.

## Revendications

1. Nécessaire (10 ; 210 ; 240 ; 250 ; 310) de prélèvement et de réinjection d'un fluide, **caractérisé en ce qu'il** comporte
- un dispositif (12) de prélèvement et de réinjection d'un fluide, le dispositif (12) comprenant :
* un corps creux (22) délimitant une cavité (40) de réception du fluide, la cavité de réception (40) débouchant par une ouverture proximale (46) et par au moins une ouverture distale (48) de prélèvement et/ou de réinjection ;
* un piston (24), monté mobile dans la cavité de réception (40) pour obturer l'ouverture proximale (46) ;
le dispositif (12) comportant un ensemble de distribution (26) sélective d'une partie proximale du fluide située dans la cavité de réception (40), l'ensemble de distribution (26) comportant un canal (70) de circulation de fluide ménagé à travers le piston (24), le canal de circulation (70) débouchant dans la cavité de réception (40) ; le nécessaire (10 ; 210 ; 240 ; 250 ; 310) comportant :
- un injecteur (16) comportant un support (130) de retenue du corps creux (22) du dispositif (12), et au moins un organe d'actionnement (126) du piston (24), monté mobile par rapport au support (130), l'organe d'actionnement (126) étant destiné à coopérer avec le piston (24) pour déplacer le piston (24) dans la cavité de réception (40).

2. Nécessaire (240) selon la revendication 1, **caractérisé en ce que** le piston (24) comporte un corps distal (61) d'obturation de la cavité (40) et une tige d'actionnement (242) du corps, le canal de circulation (70) étant ménagé à travers le corps distal (61) et à travers la tige (242).

3. Nécessaire (10 ; 210 ; 240 ; 250 ; 310) selon l'une quelconque des revendications précédentes, **caractérisé en ce que** l'ensemble de distribution (26) comporte un piquage proximal (72) de distribution raccordé au canal de circulation (70) à l'opposé de la cavité de réception (40), le piquage proximal (72) étant déplaçable conjointement avec le piston (24).

4. Nécessaire (210 ; 240) selon l'une quelconque des revendications précédentes, **caractérisé en ce que** le dispositif (12) comporte un raccord auxiliaire (170) de mise sous vide de la cavité de réception (40), le raccord auxiliaire (170) débouchant dans la cavité de réception (40) à l'écart de l'ouverture distale (48).

5. Nécessaire (10 ; 210 ; 240 ; 250 ; 310) selon l'une quelconque des revendications précédentes, **caractérisé en ce que** le dispositif (12) comporte une conduite (32) de mise sous vide destinée à être raccordée d'une part, à la cavité de réception (40) et d'autre part, à une source (16) de vide.

6. Nécessaire (10 ; 210 ; 240 ; 250 ; 310) selon l'une quelconque des revendications précédentes, **caractérisé en ce que** le dispositif (12) comporte au moins une conduite (34, 36) amovible de prélèvement et/ou de réinjection destinée à être raccordée à la cavité de réception (40) à travers l'ensemble de distribution (26).

7. Nécessaire (10 ; 210 ; 240 ; 250 ; 310) selon l'une quelconque des revendications précédentes, **caractérisé en ce que** l'extrémité proximale du corps creux (22) est munie de moyens de verrouillage (52) propres à être reçus dans des moyens de blocage complémentaire d'une centrifugeuse (18).

8. Nécessaire (310) selon l'une quelconque des revendications précédentes, **caractérisé en ce que** le dispositif (12) comporte une conduite (36) de réinjection destinée être raccordée à la cavité de réception (40), le nécessaire (310) comprenant un dispositif additionnel (312) de régulation de débit, le dispositif additionnel (312) comportant un corps creux additionnel (314) délimitant un volume intermédiaire (316), un piston (318) actionnable manuellement, disposé dans le corps creux additionnel (314), pour obturer vers l'aval le volume intermédiaire (316), la conduite de réinjection (36) étant raccordée en amont à la cavité de réception (40) et en aval au volume intermédiaire (316), avantageusement à travers le piston actionnable manuellement (318).

9. Nécessaire (310) selon la revendication 8, **caractérisé en ce que** le dispositif additionnel de régulation de débit (312) comporte un organe (319) de blocage en position du piston actionnable manuellement (318).

10. Nécessaire (10 ; 210 ; 240 ; 250 ; 310) selon l'une quelconque des revendications précédentes, **caractérisé en ce qu'il** comporte une centrifugeuse (18), la centrifugeuse (18) comportant un berceau (157) de réception du dispositif de prélèvement (12).

11. Injecteur (14) destiné à recevoir un dispositif (12) de prélèvement et de réinjection de fluide, le dispositif (12) comprenant :
* un corps creux (22) délimitant une cavité (40) de réception du fluide, la cavité de réception (40) débouchant par une ouverture proximale (46) et par au moins une ouverture distale (48) de prélèvement et/ou de réinjection ;
* un piston (24), monté mobile dans la cavité de réception (40) pour obturer l'ouverture proximale (46) ;
le dispositif (12) comportant un ensemble de distribution (26) sélective d'une partie proximale du fluide située dans la cavité de réception (40), l'ensemble de distribution (26) comportant un canal (70) de circulation de fluide ménagé à travers le piston (24), le canal de circulation (70) débouchant dans la cavité de réception (40) ;
, l'injecteur (14) comprenant :
- un support (130) de retenue du corps creux (22) du dispositif (12);
- un organe d'actionnement (126) monté mobile par rapport au support (130), l'organe d'actionnement (126) étant destiné à coopérer avec le piston (24) pour déplacer le piston (24) dans la cavité de réception (40) ;
**caractérisé en ce que** l'organe d'actionnement (126) comporte un logement (148) de réception de l'ensemble de distribution et/ou d'une conduite amovible (32, 34, 36) destinée à être raccordée au canal (70) de circulation de fluide.

12. Injecteur (14) selon la revendication 11, **caractérisé en ce que** l'organe d'actionnement (126) comporte une tige (146) mobile axialement à travers le support (130), le logement (148) débouchant axialement dans une surface (150) d'appui sur le piston (24) et débouchant transversalement à travers une surface latérale de la tige (146).

13. Procédé non thérapeutique de traitement cosmétique d'un être vivant, comprenant les étapes suivantes :
- fourniture d'un nécessaire (10 ; 210 ; 240 ; 250 ; 310) selon l'une quelconque des revendications 1 à 10, la cavité de réception (40) du corps (22) contenant un fluide non thérapeutique ;
- évacuation sélective d'une partie proximale du fluide présent dans la cavité de réception (40), le piston (24) obturant l'ouverture proximale (46), indépendamment d'une partie distale du fluide située entre la partie proximale et l'ouverture distale (48) par l'intermédiaire de l'ensemble de distribution (26), la partie proximale du fluide étant évacuée à travers le canal d'évacuation (70) ménagé dans le piston (24).

14. Procédé selon la revendication 13, **caractérisé en ce qu'il** comporte une étape de séparation du fluide contenu dans la cavité de réception (40) en une première phase proximale (164A) et en au moins une deuxième phase distale (164B), le procédé comprenant les étapes suivantes :
- évacuation de la première phase proximale (164A) hors de la cavité de réception (40) à travers l'ensemble de distribution (26) sans évacuer la deuxième phase distale (164B).

15. Procédé selon l'une quelconque des revendications 13 ou 14, **caractérisé en ce que** le dispositif (12) comporte une conduite (36) de réinjection destinée être raccordée à la cavité de réception (40), le nécessaire (310) comprenant un dispositif additionnel (312) de régulation de débit, le dispositif additionnel (312) comportant un corps creux additionnel (314) délimitant un volume intermédiaire (316), un piston (318) actionnable manuellement, disposé dans le corps creux additionnel (314), pour obturer vers l'aval le volume intermédiaire (316), la conduite de réinjection (36) étant raccordée en amont à la cavité de réception (40) et en aval au volume intermédiaire (316), avantageusement à travers le piston actionnable manuellement (318) ;
le procédé comprenant une étape d'activation de l'injecteur (14) pour amener du fluide contenu dans la cavité de réception (40) du corps creux (22) dans le volume intermédiaire (316) et une étape d'actionnement manuel du piston (318) dans le corps creux additionnel (314) pour modifier le débit de fluide extrait hors du dispositif additionnel de régulation de débit (312).

16. Procédé selon la revendication 15, **caractérisé en ce que** l'étape d'actionnement manuel comporte un déplacement du piston actionnable manuellement (318) dans le corps creux additionnel (314) pour réduire le volume intermédiaire (316) afin d'augmenter le débit de fluide extrait hors du corps creux additionnel (114) ou un déplacement du piston actionnable manuellement (318) dans le corps creux additionnel (114) pour augmenter le volume intermédiaire (316) afin de diminuer le débit de fluide extrait hors du corps creux additionnel (114).
